# EUROPEAN PATENT APPLICATION

(11) **EP 2 848 217 A1**
(43) Date of publication of application: **18.03.2015**
(21) Application number: 14184058.7
(22) Date of filing: 09.09.2014
(51) Int. Cl.: A61B 17/3203, B26F 3/00, A61B 17/00

(54) **Liquid ejection device and medical device**

(30) Priority: 11.09.2013 JP 2013188261; 01.04.2014 JP 2014075011
(71) Applicant: Seiko Epson Corporation, Tokyo 163-0811 (JP)
(72) Inventor: Uchida, Kazuaki, Suwa-shi Nagano, 392-8502 (JP); Kojima, Hideki, Suwa-shi Nagano, 392-8502 (JP); Sekino, Hirokazu, Suwa-shi Nagano, 392-8502 (JP); Seto, Takeshi, Suwa-shi Nagano, 392-8502 (JP)
(74) Representative: Hoffmann Eitle

(57) **Abstract**

A liquid injection device includes a liquid injection mechanism including a liquid chamber and an air bubble generation unit provided in the liquid chamber and generating air bubbles, and a control unit that changes a drive signal for driving the air bubble generation unit in response to a movement speed of the liquid injection mechanism.

## Description

### BACKGROUND

### 1. Technical Field

The present invention relates to injection of a liquid.

### 2. Related Art

In a liquid injection device used as a medical device, a method of measuring acceleration of an injection port and selecting a mode of liquid injection based on the acceleration is known (e.g., Patent Document 1 (JP-A-2012-143374)).

The related art is advantageous in exhibition of incision or excision performance according to intentions of an operator by switching an injection mode depending on the movement speed of the injection port. The inventors have further improved the technology and found a method of preferably changing the performance of incision or the like in response to the movement speed of the injection port. In addition, downsizing, lower cost, resource saving, facilitation of manufacture, improvement in usability of the device, etc. have been desired. The inventors have attempted to solve the problems.

### SUMMARY

An advantage of some aspects of the invention is to solve at least one of the problems described above, and the invention can be implemented as the following forms.

(1) An aspect of the invention provides a liquid injection device. The liquid injection device includes a liquid injection mechanism having a liquid chamber and an air bubble generation unit provided in the liquid chamber and generating air bubbles, and a control unit that changes a drive signal for driving the air bubble generation unit in response to a movement speed of the liquid injection mechanism. According to this aspect, the drive signal is changed in response to the movement speed, and thereby, the condition of the air bubble generation unit may be adjusted in response to the movement speed and the depth of excision may be stabilized.

(2) In the aspect described above, the air bubble generation unit may adjust a volume of air bubbles generated per unit time in response to at least one of a voltage, a current, power, and a duty of the drive signal. In this case, the control unit may set the drive signal to a first state when the movement speed is a first speed, and set a voltage of the drive signal to a second state in which the volume of the generated air bubbles is larger than that in the first state when the movement speed is a second speed higher than the first speed. According to this aspect, the volume of the air bubbles generated per unit time may be easily controlled. This is because the volume of the air bubbles generated per unit time may be relatively easily increased.

(3) In the aspect described above, the control unit may change a frequency of the drive signal in response to the movement speed. According to this aspect, the volume of the air bubbles generated per unit time may be adjusted using other methods than changing the voltage, the current, the power, and the duty.

(4) In the aspect described above, the control unit may set the frequency of the drive signal to a first frequency when the voltage is in the first state or the second state, and set the frequency of the drive signal to a second frequency higher than the first frequency when the drive signal is in a third state in which the volume of the generated air bubbles is larger than that in the second state. According to this aspect, when the voltage is a first or second voltage, the output is controlled by changing of the voltage without changing of the frequency of the drive signal, and thereby, the values of the first and second voltages may be easily determined.

(5) In the aspect described above, the control unit may set the drive signal to the third state and sets the frequency of the drive signal to the second frequency when the movement speed is a third speed higher than the second speed, and set the drive signal to the third state and sets the frequency of the drive signal to a third frequency higher than the second frequency when the movement speed is a fourth speed higher than the third speed. According to this aspect, when the movement speed is the third or fourth speed, the output is controlled by changing of the frequency without changing of the voltage of the drive signal, and thereby, the values of the second and third frequencies may be easily determined.

(6) In the aspect described above, a liquid feed unit that feeds a liquid to the liquid chamber at a flow rate set by the control unit is provided, and the control unit sets the flow rate to a first flow rate when the movement speed is the first speed and sets the flow rate to a second flow rate higher than the first flow rate when the movement speed is the second speed. According to this aspect, the flow rate to be fed may be properly set.

(7) In the aspect described above, the control unit may change the state and the frequency of the drive signal in response to the movement speed. According to this aspect, the generation of the air bubbles by the air bubble generation unit may be changed by the state and the frequency of the drive signal.

The aspects of the invention may be realized in various another forms. For example, the aspects of the invention may be realized in forms of a liquid injection method, a medical device, a surgery method, programs for realization of the methods, memory media storing the programs, etc.

### BRIEF DESCRIPTION OF THE DRAWINGS

The invention will be described with reference to the accompanying drawings, wherein like numbers reference like elements.
Fig. 1 is a configuration diagram of a liquid injection device of a first embodiment.
Fig. 2 is a sectional view showing inside of a liquid injection mechanism of the first embodiment.
Fig. 3 is a graph showing a waveform of a drive signal.
Fig. 4 is a flowchart showing injection processing of the first embodiment.
Figs. 5A and 5B are graphs showing relations between a drive voltage and a drive frequency and a movement speed.
Fig. 6 is a graph showing a relation between the drive frequency and the drive voltage.
Fig. 7 is a flowchart showing injection processing of a second embodiment.
Fig. 8 is a configuration diagram of a liquid injection device of a third embodiment.
Fig. 9 is a sectional view showing inside of a liquid injection mechanism of the third embodiment.

### DESCRIPTION OF EXEMPLARY EMBODIMENTS

### A. First Embodiment:

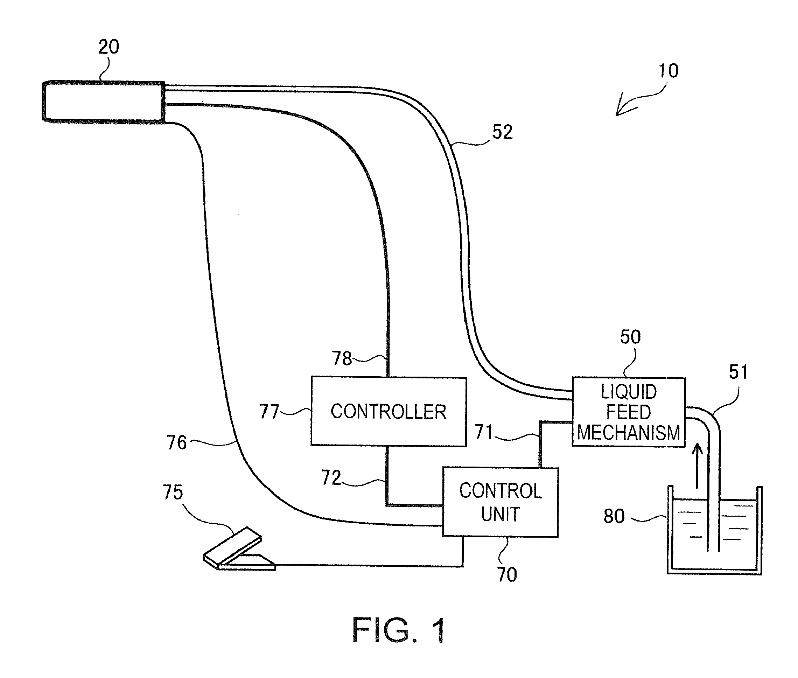

An embodiment of the invention will be explained. Fig. 1 shows a configuration of a liquid injection device 10 as the first embodiment. The liquid injection device 10 is a medical device used in medical institutions, and has a function of injecting a liquid to an affected part to incise or excise the affected part.

The liquid injection device 10 includes a liquid injection mechanism 20, a liquid feed mechanism 50, a control unit 70, a controller 77, and a liquid container 80. The liquid feed mechanism 50 and the liquid container 80 are connected to each other by a connecting tube 51. The liquid feed mechanism 50 and the liquid injection mechanism 20 are connected to each other by a liquid feed channel 52. The connecting tube 51 and the liquid feed channel 52 are formed using resin. The connecting tube 51 and the liquid feed channel 52 may be formed using other materials (e. g. , metal) than resin.

The liquid container 80 stores saline. In place of saline, pure water or chemical solution may be stored therein. The liquid feed mechanism 50 feeds a liquid suctioned from the liquid container 80 by driving of a pump inside via the connecting tube 51 to the liquid injection mechanism 20 via the liquid feed channel 52.

The liquid injection mechanism 20 is a tool operated in hand by a user of the liquid injection device 10. The user applies the liquid intermittently injected from the liquid injection mechanism 20 to the affected part, and thereby, incises or excises the affected part.

The control unit 70 controls the liquid feed mechanism 50 via a control cable 71, and thereby, controls a flow rate of the liquid fed to the liquid injection mechanism 20 (hereinafter, referred to as "feed flow rate"). A foot switch 75 is connected to the control unit 70. When the user turns on the foot switch 75, the control unit 70 controls the liquid feed mechanism 50 to execute feed of the liquid to the liquid injection mechanism 20 and transmit a drive signal to the controller 77 via a signal cable 72.

The controller 77 supplies power to the liquid injection mechanism 20 via a signal cable 78 in response to the drive signal. By the power supply, as will be described later, air bubbles are generated in an air bubble generation unit, and the liquid is injected from the liquid injection mechanism 20.

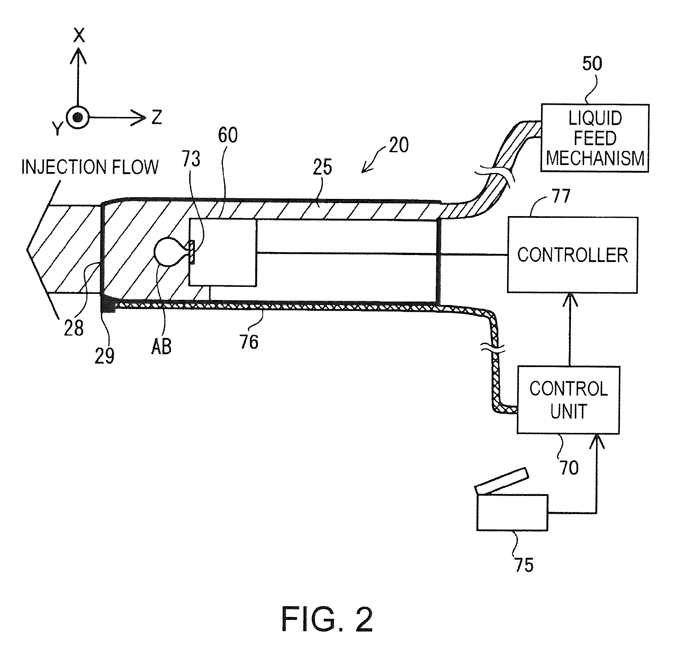

Fig. 2 is a sectional view showing inside of the liquid injection mechanism 20. The liquid injection mechanism 20 forms a liquid chamber 25 inside. The liquid chamber 25 has openings on both a posterior end and an anterior end and the opening of the posterior end is connected to the liquid feed mechanism 50 via the liquid feed channel 52. Accordingly, the liquid chamber 25 is filled with the liquid fed from the liquid feed mechanism 50. On the other hand, the opening of the anterior end of the liquid chamber 25 is formed as an injection port 28.

An air bubble generation unit 60 is provided in the liquid chamber 25 of the liquid injection mechanism 20. A heater 73 that generates heat by energization is provided on one end surface of the air bubble generation unit 60. When a voltage is applied to the heater 73 by a voltage signal output from the controller 77, the heater 73 promptly generates heat. The heat of the heater 73 is absorbed by the liquid filling the liquid injection mechanism 20 and the liquid is vaporized. In the first embodiment, the heater 73 is intermittently energized, and the vaporization intermittently occurs (if the liquid is water, it instantaneously boils). The intermittently occurring vaporization instantaneously increases the pressure of the liquid within the liquid injection mechanism 20. The instantaneously increased pressure injects the liquid from the injection port 28. Incidentally, Fig. 2 shows an operation principle of the liquid injection mechanism 20, and the actual injection port 28 is formed to be thinner to emit a thin flow (pulsating flow) for surgery. When the heater 73 is energized and the liquid is vaporized, the liquid within the liquid chamber 25 subjected to the pressure spouts as a pulsating flow from the injection port 28.

The liquid injection mechanism 20 includes an acceleration sensor 29. The acceleration sensor 29 is a piezoresistive triaxial acceleration sensor. As shown in Fig. 2, the acceleration sensor 29 is provided near the injection port 28 and outside of a casing of the liquid injection mechanism 20. Measurement results are input to the control unit 70 via an acceleration sensor cable 76. The acceleration sensor cable 76 is fixed to the outside of the casing of the liquid injection mechanism 20 by bonding from the connecting part to the acceleration sensor cable 76 to the posterior end of the liquid injection mechanism 20 (the opposite side to the injection port 28).

The three axes as the measuring objects of the acceleration sensor 29 are respective axes of XYZ shown in Fig. 2. The Z-axis is in parallel to the longitudinal axis directions of the liquid injection mechanism 20, i.e., in parallel to the injection direction of the liquid, and the direction in which the liquid is injected is a negative direction. The X-axis is orthogonal to the Z-axis and a predetermined direction is a positive direction. The predetermined direction is upward in the vertical direction when the Z-axis is directed to be horizontal and the acceleration sensor 29 is located immediately below as shown in Fig. 2. The Y-axis is defined by the right-handed system with reference to the X-axis and the Z-axis.

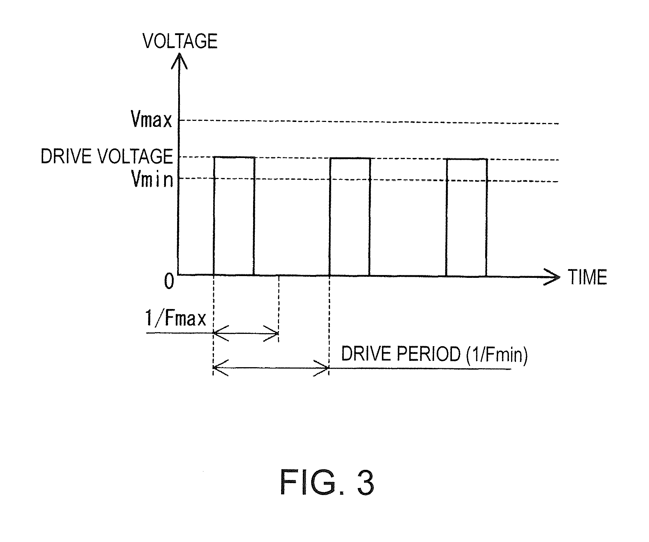

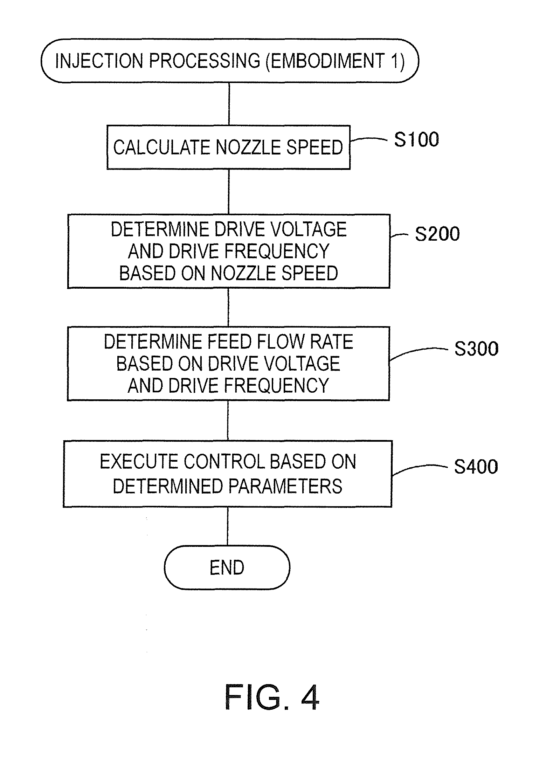

Fig. 3 is a graph showing a waveform of the drive signal. The drive signal is input to the controller 77 for heating the heater 73 as described above. The vertical axis indicates the voltage and the horizontal axis indicates time. The waveform of the drive signal in the embodiment is pulsed as shown in Fig 3. The maximum voltage of each pulsed wave (hereinafter, referred to as "drive voltage") and the frequency of the pulsed wave (hereinafter, referred to as "drive frequency") change depending on the injection processing, which will be described later with Fig. 4.

Fig. 3 exemplifies the case where the drive voltage takes a value between the maximum value (Vmax) and the minimum value (Vmin) and the drive period takes the maximum value, i. e. , the drive frequency takes the minimum value (Fmin). When the drive voltage is larger, the controller 77 controls energization so that the amount of power supplied to the heater 73 by single heating may be larger. On the other hand, when the drive frequency is larger, the controller 77 controls energization so that the number of times of heating of the heater 73 per unit time may be larger. In either case, the amount of power for heating the heater 73 (energy per unit time) is larger. When the amount of power for heating the heater 73 is larger, excision performance is improved as will be described later.

Fig. 4 is a flowchart showing injection processing. The injection processing is repeatedly executed by the control unit 70 while the foot switch 75 is pushed. First, the speed S of the injection port 28 is calculated (step S100). The speed S here refers to an absolute value of the speed in the XY-plane. That is, the speed is an absolute value of the velocity when the velocity in the Z-axis direction is ignored. The speed S is calculated based on the acceleration on the three axes measured by the acceleration sensor 29.

The speed S is calculated as a parameter having an effect on the depth of the excision of the affected part. This is because the excision performance acting on the respective local areas of the affected part per unit time is influenced by the relative speed of the injection port 28 and the affected part. Accordingly, in consideration of the case where the affected part moves with breathing of a patient or the like, the speed S may be treated as the movement speed of the affected part and the injection port 28, however, in the embodiment, on the assumption that the affected part remains stationary, the speed S is treated as the movement speed of the affected part and the injection port 28.

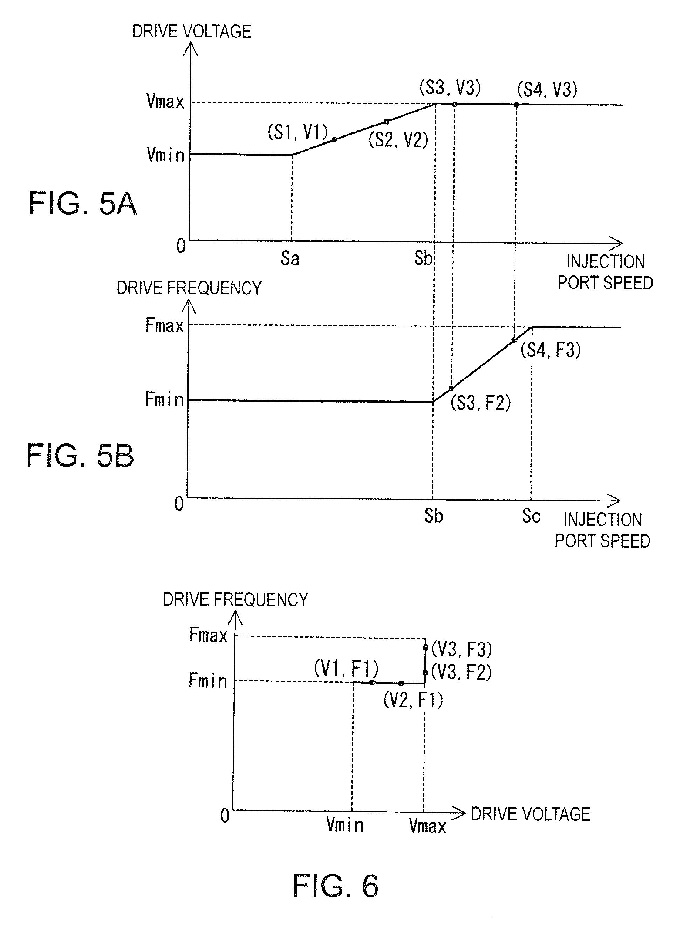

Subsequently, the drive voltage and the drive frequency are determined based on the calculated speed S (step S200). Figs. 5A and 5B are graphs showing relations between the drive voltage and the drive frequency and the speeds. Fig. 5A shows the drive voltage and Fig. 5B shows the drive frequency on the vertical axes. The horizontal axes indicate the speed S in common and the scales are the same in Figs. 5A and 5B.

As shown in Figs. 5A and 5B, in the respective speed ranges of Sa ≤ speed S ≤ Sb, Sb ≤ speed S ≤ Sc, the parameters with values to be changed are different. That is, Sa, Sb, and Sc are speeds predetermined as threshold values for switching the parameters to be changed.

If S ≤ Sa, the drive voltage is fixed to Vmin as the minimum value and the drive frequency is fixed to Fmin as the minimum value. When the parameters are set as described above, the excision performance is the lowest.

If Sa ≤ speed S ≤ Sb, the drive frequency is fixed to Fmin, and the drive voltage linearly increases with respect to the increase of the speed S. If speed S = Sb, the drive voltage is set to Vmax as the maximum value. Vmin is set so that the excision performance may not be too low. Vmax is set so that the heater 73 may not be excessively heated. When the drive voltage is larger, the amount of power per single energization to the heater 73 is larger, and the pressure fluctuations within the liquid injection mechanism 20 are larger. As a result, the liquid is energetically injected and the excision performance is greater, and, even when the speed S is higher, the depth of excision is stable.

If Sb ≤ speed S ≤ Sc, the drive voltage is fixed to Vmax, and the drive frequency linearly increases with respect to the increase of the speed S. If speed S = Sc, the drive frequency is set to Fmax as the maximum value. Fmin is set so that the excision performance may not be too low. Fmax is set so that the heater 73 may not be excessively heated. When the drive frequency is larger, the number of times of injection per unit time is larger. As a result, the excision performance is larger, and, even when the speed S is higher, the depth of excision is stable. If the period of the energization is shorter, the time taken for cooling the heater 73 at a high temperature by energization with the liquid is not sufficiently secured. The upper limit of the drive frequency is determined in consideration of the time taken for cooling the heater 73.

The drive voltage and the drive frequency are determined as described above, and then, the feed flow rate is determined (step S300). The feed flow rate is determined to be a sufficient value for replenishment of the liquid to be intermittently injected. When the value is larger in either case of the drive voltage or the drive frequency, the amount of liquid injected per unit time increases. Therefore, at least one of the drive voltage and the drive frequency is increased, and the feed flow rate is increased. Finally, the control is executed based on the determined drive voltage, drive frequency, and feed flow rate (step S400).

As described above, the drive voltage is changed so that the excision performance may be improved with the increase of the speed S, and thereby, the drive voltage applied to the heater 73 may be changed and the depth of excision may be stabilized. The amount of power supplied to the heater 73 may be greatly controlled by the drive voltage, and is preferable as a parameter for controlling generation of air bubbles. Further, when the drive voltage reaches the maximum value, the drive frequency is changed, and thereby, the depth of excision may be stabilized.

Fig. 6 is a graph showing a relation between the drive frequency and the drive voltage. As described above, the drive frequency does not change in the range of the speed S (Sa ≤ speed S ≤ Sb) in which the drive voltage changes, but changes in the range of the speed S (Sb ≤ speed S ≤ Sc) in which the drive voltage is Vmax. The ranges of the speed S in which the drive voltage and the drive frequency are changed are separated, and thus, the values of the drive voltage and the drive frequency are easily determined in the respective speed ranges.

As shown in Figs. 5A to 6, S1 to S4 are examples of first to fourth speeds, V1 to V3 are examples of first to third voltages, and F1 to F3 are examples of first to third frequencies in the appended claims. The heater 73 and the controller 77 are examples of an air bubble generation unit in the appended claims.

### B. Second Embodiment:

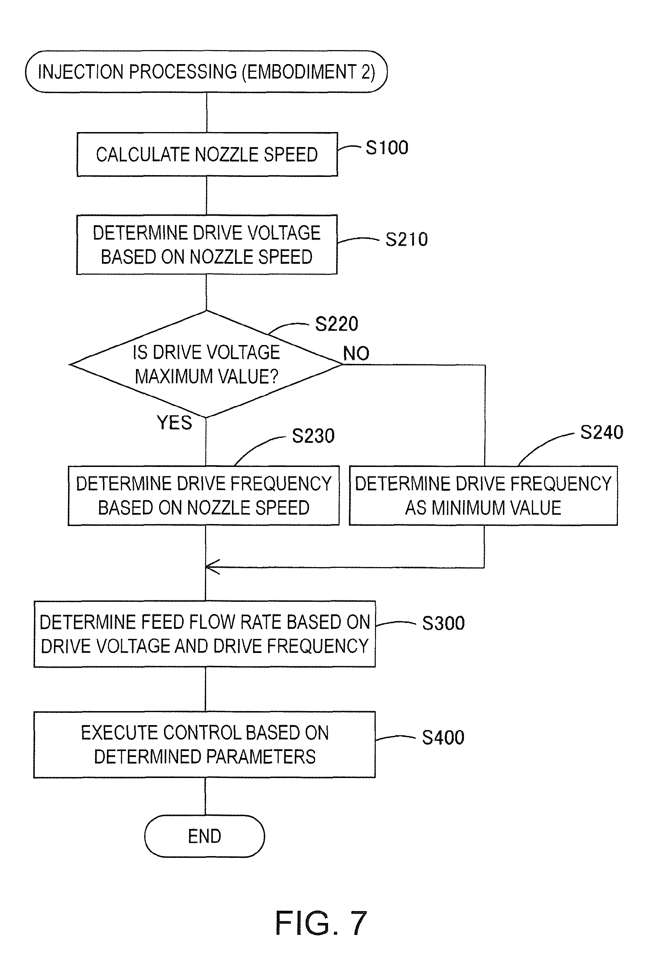

Next, the second embodiment of the invention will be explained. The second embodiment executes injection processing shown in Fig. 7 in place of the injection processing shown in Fig. 4. The hardware configuration is the same as that of Embodiment 1 and the explanation will be omitted. Step S100, step S300, and step S400 in the injection processing in Embodiment 2 are the same as those of Embodiment 1 and the explanation will be omitted. In Embodiment 2, step S210 to step S240 are executed in place of step S200 of Embodiment 1.

The speed S is calculated (step S100), and then, the drive voltage is determined based on the calculated speed S (step S210). The method of determining the drive voltage is the same as that of Embodiment 1. The drive voltage is fixed to Vmin if speed S ≤ Sa, linearly increases with the increase of the speed S if Sa ≤ speed S ≤ Sb, and fixed to Vmax if speed Sb ≤ speed S.

Then, whether or not the drive voltage is set to the maximum value (Vmax) is determined (step S220). If the drive voltage is set to a value less than the maximum value (step S220, NO), the drive frequency is set to the minimum value (Fmin) (step S240). The fact that the drive voltage is set to a value less than the maximum value means that the excision performance may be further improved by changing of the drive voltage. Accordingly, it is not necessary to improve the excision performance by changing of the value of the drive frequency, and the drive frequency is set to the minimum value.

On the other hand, if the drive voltage is set to the maximum value (step S220, YES), the drive frequency is determined based on the speed S (step S230). The method of determining the drive frequency is the same as that of Embodiment 1. The drive frequency is fixed to Fmin if speed S ≤ Sb, linearly increases with the increase of the speed S if Sb ≤ speed S ≤ Sc, and fixed to Fmax if speed Sc ≤ speed S.

The fact that the drive voltage is set to the maximum value means that the excision performance may not be further improved by changing of the drive voltage. Accordingly, in order to improve the excision performance by changing the value of the drive frequency, step S230 is executed. According to Embodiment 2, the same control result as that of Embodiment 1 may be obtained.

### C. Third Embodiment:

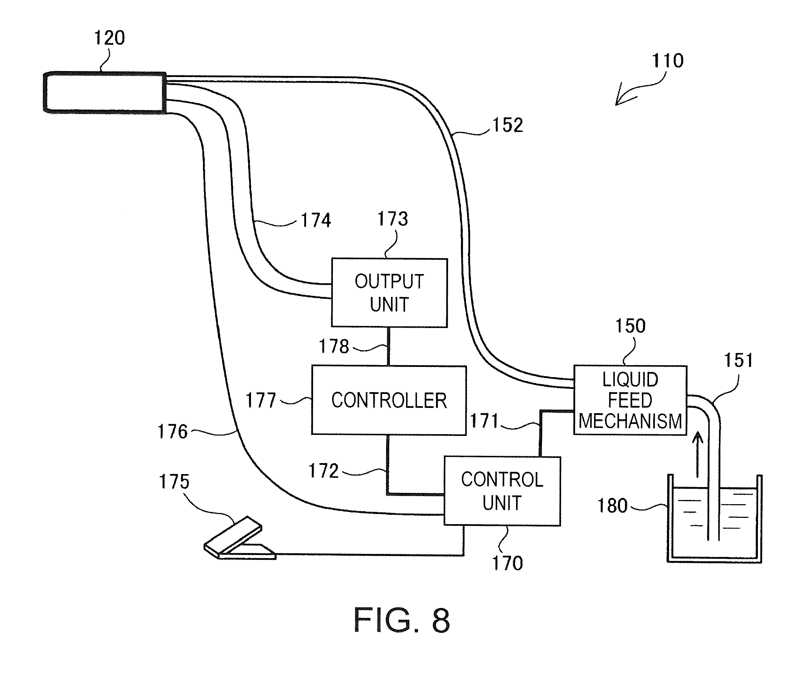

The third embodiment of the invention will be explained. Fig. 8 is a schematic configuration diagram of a liquid injection device 110 of the third embodiment. The liquid injection device 110 is also a medical device used in medical institutions, and has a function of injecting a liquid to an affected part to incise or excise the affected part.

The liquid injection device 110 includes a liquid injection mechanism 120, a liquid feed mechanism 150, a control unit 170, an output unit 173, a controller 177, and a liquid container 180. The liquid feed mechanism 150 and the liquid container 180 are connected to each other by a connecting tube 151. The liquid feed mechanism 150 and the liquid injection mechanism 120 are connected to each other by a liquid feed channel 152. The connecting tube 151 and the liquid feed channel 152 are formed using resin. The connecting tube 151 and the liquid feed channel 152 may be formed using other materials (e.g., metal) than resin.

The liquid container 180 stores saline. In place of saline, pure water or chemical solution may be stored. The liquid feed mechanism 150 feeds a liquid suctioned from the liquid container 180 by driving of a pump inside via the connecting tube 151 to the liquid injection mechanism 120 via the liquid feed channel 152.

The liquid injection mechanism 120 is a tool operated in hand by a user of the liquid injection device 110. The user applies the liquid intermittently injected from the liquid injection mechanism 120 to the affected part, and thereby, incises or excises the affected part.

The control unit 170 controls the liquid feed mechanism 150 via a control cable 171, and thereby, controls a flow rate of the liquid fed to the liquid injection mechanism 120 (hereinafter, referred to as "feed flow rate"). A foot switch 175 is connected to the control unit 170. When the user turns on the foot switch 175, the control unit 170 controls the liquid feed mechanism 150 to execute feed of the liquid to the liquid injection mechanism 120 and transmit a drive signal to the controller 177 via a signal cable 172.

In order to output optical maser in response to the drive signal, the controller 177 outputs a control signal to the output unit 173 via a signal cable 178. The output unit 173 includes holmium:YAG optical maser, and outputs optical maser according to the control signal. The wavelength of the optical maser is 2.06 µm. The output optical maser passes through an optical maser cable 174 formed using an optical fiber and is guided into the liquid injection mechanism 120.

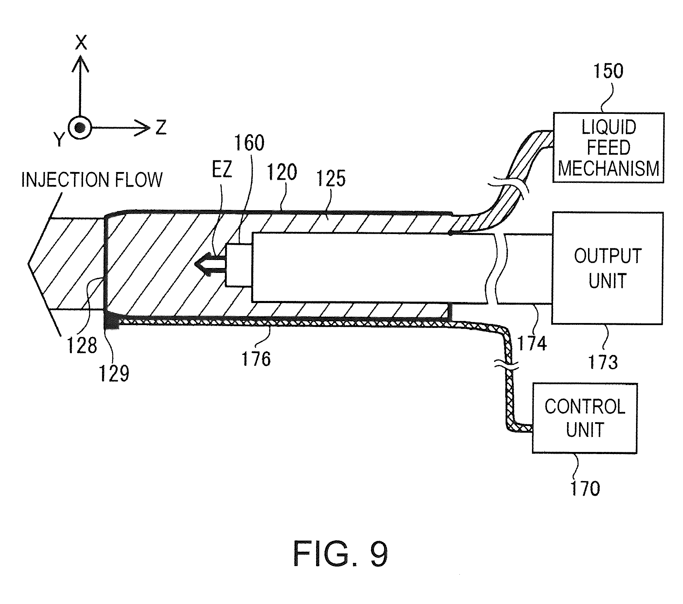

Fig. 9 is a sectional view showing inside of the liquid injection mechanism 120. The liquid injection mechanism 120 forms a liquid chamber 125 inside. The liquid chamber 125 is filled with the liquid fed from the liquid feed mechanism 150. The optical maser guided by the optical maser cable 174 is emitted within the liquid injection mechanism 120. The emitted optical maser is absorbed by the liquid filling the liquid injection mechanism 120. The part in which the optical maser is absorbed is shown as an air bubble generation unit 160 in Fig. 8. The liquid that has absorbed the optical maser is vaporized by the absorbed energy and forms air bubbles. In the embodiment, the optical maser is intermittently output, and the vaporization intermittently occurs. The intermittently generated air bubbles instantaneously increase the pressure of the liquid within the liquid injection mechanism 120. The instantaneously increased pressure injects the liquid from an injection port 128. The condition in which the pressure acts on the liquid by the generated air bubbles in the direction of the injection port 128 is shown by an arrow EZ in Fig. 9.

The liquid injection mechanism 120 includes an acceleration sensor 129. The acceleration sensor 129 is a piezoresistive triaxial acceleration sensor. As shown in Fig. 9, the acceleration sensor 129 is provided near the injection port 128 and outside of a casing of the liquid injection mechanism 120. Measurement results are input to the control unit 170 via an acceleration sensor cable 176. The acceleration sensor cable 176 is fixed to the outside of the casing of the liquid injection mechanism 120 by bonding from the connecting part to the acceleration sensor cable 176 to the posterior end of the liquid injection mechanism 120 (the opposite side to the injection port 128).

The three axes as the measuring objects of the acceleration sensor 129 are respective axes of XYZ shown in Fig. 9. The Z-axis is in parallel to the longitudinal axis directions of the liquid injection mechanism 120, i.e., in parallel to the injection direction of the liquid, and the direction in which the liquid is injected is a negative direction. The X-axis is orthogonal to the Z-axis and a predetermined direction is a positive direction. In the third embodiment, the relation among the respective axes is the same as that of the first embodiment such that the Y-axis is defined by the right-handed system with reference to the X-axis and the Z-axis.

In the third embodiment, the air bubbles are generated in the liquid chamber 125 by energy injection using the optical maser as described above, in place of heating by the heater 73 of the first embodiment. When the optical maser is output from the output unit 173, the optical maser is guided by the optical fiber 174 and emitted from the end thereof. The liquid within the liquid chamber 125 absorbs the optical maser and forms air bubbles. The waveform of the drive signal with respect to the output unit 173 is the same as that shown in the graph of Fig. 3. As described above, the drive signal is input to the controller 177 for outputting the optical maser. The drive signal that intermittently operates the optical maser is output as pulsed wave. The maximum voltage of each pulsed wave (hereinafter, referred to as "drive voltage") and the frequency of the pulsed wave (hereinafter, referred to as "drive frequency") change depending on injection processing.

The injection processing in the third embodiment is the same as that of the first embodiment (Fig. 4) and the drive voltage output from the control unit 170 is the same as the drive voltage of the first embodiment (Fig. 3). The control ranges of the drive voltage and the drive frequency output from the control unit 170 are the same as those of the first embodiment (Figs. 5A and 5B). Further, the relation between the drive frequency and the drive voltage is the same as that of the first embodiment (Fig. 6). Therefore, also, in the third embodiment, when one of the drive voltage and the drive frequency output from the control unit 170 to the controller 177 is larger, the output of the emitted optical maser (energy per unit time) is larger. When the output of the emitted optical maser is larger, the excision performance is improved.

Therefore, in the third embodiment, the same advantages as those of the first embodiment may be obtained. Further, in the third embodiment, the optical maser is used for the output unit 173 and, in the air bubble generation unit 160, the liquid absorbs the energy of the optical maser and forms air bubbles. Thus, the output unit 173 as the energy generation unit is not within the liquid injection mechanism 120 and, even when the liquid injection mechanism 120 is contaminated, replacement of the output unit 173 is unnecessary.

The invention is not limited to the embodiments, examples, modified examples of the specification, and may be realized in various configurations without departing from the scope thereof. For example, the technological features in the embodiments, examples, modified examples corresponding to the technological features in the respective embodiments described in Summary of the Invention may be appropriately replaced or combined in order to solve part or all of the above described problems or achieve part or all of the above described advantages. If the technological features are not explained as essential features in the specification, they may be appropriately deleted. For example, the following features are exemplified.

The drive voltage and the drive frequency may be determined using functions.

The speed range in which the drive voltage is varied and the speed range in which the drive frequency is varied may overlap.

The waveform of the drive signal is not limited to the pulsed wave, but may be a sine curve or the like, for example.

The relations between the respective drive voltage and drive frequency and the speed of the injection port may be specified by a curve or steps.

Only one of the drive voltage and the drive frequency may be changed.

When the drive voltage is changed, not limited to the change of the maximum voltage, but voltages equal to or more than a predetermined value or voltages in a predetermined period may be changed.

At least one of the drive voltage and the drive frequency may be changed in response to the distance between the injection port and the affected part. This is because the distance between the injection port and the affected part is considered as a parameter relating to the depth of excision like the movement speed of the injection port and the affected part. Specifically, as the distance between the injection port and the affected part is larger, at least one of the drive voltage and the drive frequency may be changed for improvement of the excision performance.

The output of the optical maser may be adjusted by changing of a pulse width. The pulse width is a time in which the drive signal reaches the maximum voltage.

The speed of the injection port may be calculated using image processing. For example, the speed of the injection port may be calculated by providing a marker near the injection port and capturing the movement of the marker with a camera.

When a robot operates the liquid injection device, it is not necessary to calculate the speed of the injection port because the robot may grasp the speed. The grasped value may be used.

The movement speed of the injection port may be calculated in consideration of the movement speed of the affected part. The measurement of the movement speed of the affected part may be realized by prediction or measurement of the movement due to breathing and pulsing.

Further, the movement speed may be detected not only in the injection port but also in a part moving with the movement of the injection port. The movement speed of the liquid injection mechanism may be detected.

The type of the acceleration sensor may be a capacitance type or heat detection type. Further, a sensor that may indirectly or directly detect the speed, not the acceleration may be employed.

The liquid injection device may be used for others than the medical device.

For example, the liquid injection device may be used for a cleansing device that removes dirt with the injected liquid.

The liquid injection device may be used for a drawing device that draws lines etc. with the injected liquid.

To generate air bubbles in the air bubble generation unit, other configurations than the heater of the first, second embodiments or the optical maser of the third embodiment may be employed. For example, microwave or the like may be used. Further, for the heater, not limited to a metal type including nichrome and tungsten, but a ceramic type may be used.

Further, the type of the optical maser may be another solid type than holmium: YAG or a semiconductor type, a liquid type, or a gas type optical maser may be used.

When the kind of the liquid to be injected is changed, the wavelength of the optical maser or the like may be changed to a wavelength that is easily absorbed by the changed liquid.

The method of feeding the liquid is not limited to that using driving of the pump, but may be a method using the liquid's own weight, for example.

## Claims

1. A liquid injection device comprising:
a liquid injection mechanism including a liquid chamber and an air bubble generation unit provided in the liquid chamber and generating air bubbles; and
a control unit that changes a drive signal for driving the air bubble generation unit in response to a movement speed of the liquid injection mechanism.

2. The liquid injection device according to claim 1, wherein the air bubble generation unit can adjust a volume of air bubbles generated per unit time in response to at least one of a voltage, a current, power, and a duty of the drive signal, and
the control unit sets the drive signal to a first state when the movement speed is a first speed, and sets a voltage of the drive signal to a second state in which the volume of the generated air bubbles is larger than that in the first state when the movement speed is a second speed higher than the first speed.

3. The liquid injection device according to claim 2, wherein the control unit changes a frequency of the drive signal in response to the movement speed.

4. The liquid injection device according to claim 3, wherein the control unit sets the frequency of the drive signal to a first frequency when the voltage is in the first state or the second state, and sets the frequency of the drive signal to a second frequency higher than the first frequency when the drive signal is in a third state in which the volume of the generated air bubbles is larger than that in the second state.

5. The liquid injection device according to claim 4, wherein the control unit sets the drive signal to the third state and sets the frequency of the drive signal to the second frequency when the movement speed is a third speed higher than the second speed, and sets the drive signal to the third state and sets the frequency of the drive signal to a third frequency higher than the second frequency when the movement speed is a fourth speed higher than the third speed.

6. The liquid injection device according to claim 2, further comprising a liquid feed unit that feeds a liquid to the liquid chamber at a flow rate set by the control unit,
wherein the control unit sets the flow rate to a first flow rate when the movement speed is the first speed and sets the flow rate to a second flow rate higher than the first flow rate when the movement speed is the second speed.

7. The liquid injection device according to claim 1, 2 or 6, wherein the control unit changes the state and the frequency of the drive signal in response to the movement speed.

8. A medical device comprising the liquid injection device according to any one of the preceding claims.
